# EUROPEAN PATENT APPLICATION

(11) **EP 0 558 944 A2**
(43) Date of publication of application: **08.09.1993**
(21) Application number: 93101710.7
(22) Date of filing: 04.02.1993
(51) Int. Cl.: C12N 15/11, C12N 1/21, A01H 5/00, A01H 5/10, C12Q 1/68, A01N 63/02, C12N 9/00

(54) **RNA and DNA molecules for producing virus resistance**

(30) Priority: 06.02.1992 DE 4203441
(71) Applicant: Max-Planck-Gesellschaft zur Förderung der Wissenschaften e.V., D-37073 Göttingen (DE); BAYER AG, 51368 Leverkusen (DE); HOECHST AKTIENGESELLSCHAFT, D-65926 Frankfurt am Main (DE)
(72) Inventor: Loss, Peter, Dr., W-4000 Düsseldorf (DE); Schreier, Peter, Dr., W-5000 Köln (DE); Maiss, Edgar, Dr., W-3300 Braunschweig (DE); Schneider, Rudolf, Dr. c/o Hoechst AG, W-6230 Frankfurt/Main 80 (DE)
(74) Representative: VOSSIUS & PARTNER

(57) **Abstract**

The invention relates to RNA molecules which are complementary to at least one part of a viral RNA replicative intermediate and which inhibit the viral growth cycle by binding to the RNA replicative intermediate and preferably by specifically cleaving the RNA replicative intermediate, thereby achieving a reliable improvement in virus resistance in the desired organisms.

## Description

The invention substantially relates to RNA molecules being complementary to at least one part of a virus RNA replicative intermediate and inhibiting the viral growth cycle by stably binding to said RNA replicative intermediate. Furthermore, the invention relates to DNA molecules coding for said RNA molecules. Further embodiments are evident from the following description and the patent claims.

RNA viruses are viruses whose genome consists of a ribo-nucleic acid (RNA) which may be single-stranded or double-stranded. In single-stranded RNA viruses replication proceeds via a synthesis intermediate step, the so-called replicative intermediate, which is a strand being exactly complementary to the virion RNA incorporated in the virus particle.

In addition to the RNA viruses which form a complementary RNA as replicative intermediate, single-stranded DNA viruses such as the hepatitis B virus (HBV) are known which synthesize an RNA replicative intermediate during their growth cycle.

Both for procaryotes and eukaryotes naturally occurring antisense RNA molecules have been described (van der Krol et al., 1988). During the past years antisense molecules have been increasingly used against endogenous RNA molecules for the inhibition of gene expression both in plants and animals (Izant and Weintraub, 1984). These antisense RNA molecules exhibit a very high degree of complementarity to the corresponding substrate RNA molecules so that a hybrid molecule can be produced under in vivo conditions. Furthermore, antisense RNA molecules have been used against viral RNA molecules to protect plants against viral infections such as cucumber mosaic virus (Rezaian et al. 1988), potato virus X (Hemenway et al., 1988) or the tobacco mosaic virus (Powell et al., 1989). In all cases at best a protection against a systemic viral infection at very low inoculum concentrations could be achieved. Therefore, the coat protein protection (Beachy, 1990), which is increasingly used as alternative technique, presently must be considered the preferred method for achieving virus resistance in transgenic plants. According to said method the coat protein is over-expressed in the transgenic plants. A mechanism which without expression of a coat protein virtually results in a cross-protection can be observed in the presence of viral nucleic acids of a non-pathogenic or only slightly pathogenic virus whose RNA shows a certain degree of identity to the viral RNA of the pathogenic strain. The viral (+) strand or (-) strand RNA of the protective virus can interact with the complementary RNA of the pathogenic virus and can thus neutralize the latter (Palukaitis and Zaitlin, 1984). Parts of the (+) or (-) strand RNA of the protective virus could have a similar effect if they were integrated in the genome of the host plant (Palukaitis and Zaitlin, 1984).

In a further method for the inhibition of endogenous mRNA ribozymes can be used. In contrast to the above mentioned antisense RNA molecules "ribozymes" possess an enzymatic activity which allows to cleave the substrate RNA at a defined site (Cotten and Birnstiel, 1989; Steinecke et al., 1992). The basic structure of the bimolecularly used "ribozymes" is derived from the consensus structure which is found in certain viroids, virusoids and linear satellite RNAs, in the present case being monomolecular, the so-called hammerhead structure (Symons, 1989). The bond of the ribozyme requires two regions being complementary to the substrate RNA molecule which flank the cleavage site localized on the substrate RNA molecule. Between the regions of the molecule of the ribozyme hybridizing to the substrate RNA molecule there is a highly preserved structure of usually 22 nucleotides.

The bimolecular ribozyme/substrate complex is illustrated in the following:

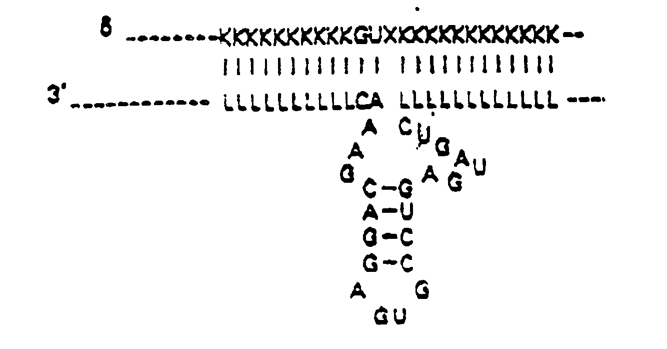

K represents any nucleotides of the substrate RNA molecule and L the corresponding complementary nucleotides of the ribozyme RNA. The other nucleotides are highly preserved. The "GUGA" hair pin structure (loop) can be replaced by other sequences since it varies also in naturally occurring satellite RNAs. In the case of the consensus sequence nucleotides C, A and U can be substituted for position X.

The substantial drawback of the use of the known antisense RNA and ribozyme techniques is the low reliability in achieving virus resistances. Therefore, these techniques so far have not gained the importance of the coat protein protection in the field of prophylaxis and therapy.

The technical problem of the present invention is therefore to provide RNA and DNA molecules with which an increased virus resistance can be achieved in the desired host organisms which substantially protects the host organism from infections.

The above technical problem is solved by the provision of the embodiments characterized in the patent claims.

The subject matter of the present invention is therefore an RNA molecule having the following features:
(a) it exhibits a sequence which is complementary to at least one part of a viral RNA replicative intermediate;
(b) it can intracellularly form a stable bond with the viral RNA replicative intermediate;
(c) it binds to a part of the viral RNA replicative intermediate which is available under intracellular conditions for such a bond; and
(d) the stable bond to the viral RNA replicative intermediate inhibits the viral growth cycle.

The substantial advantage of the RNA molecules of the present invention is the bond to viral RNA replicative intermediates whose intracellular concentration is considerably lower than that of viral mRNA transcripts and of genomic viral RNA (Nassuth and Bol, 1983) or of DNA. These viral RNA replicative intermediates being present in low intracellular concentration represent a target for the inhibition of viral growth which is easier to control. Therefore, the antiviral effect of the RNA molecules of the present invention as compared to the antiviral effect of the RNA molecules known from the prior art is increased and thus a reliable inhibition of viral growth or an increase in virus resistance of the desired host organisms is achieved.

In this context "viral RNA replicative intermediate" refers to the RNA resulting from the replication of single-stranded RNA or DNA viruses which is complementary to the virion RNA or DNA which serves to synthesize new virion RNA or DNA or subgenomic RNAs.

Furthermore, the term "stable bond" means an interaction between substrate and active molecule which is stable in the ionic environment of the cell on the basis of complementary sequences. The resulting helical stability is determined both by the length and the sequence.

The term "available for a bond under intracellular conditions" means that neither the intramolecular individual structure of the substrate molecule nor that of the antisense or ribozyme molecule should inhibit an intermolecular interaction. In order to be able to make predictions on this possibility of interaction, the thermodynamic properties can be simulated with the help of various computer programs (e.g., according to Abrahams et al., 1990).

The term "inhibition of the viral growth cycle" means that after bonding of the antisense or ribozyme molecules it is no longer possible or only to a reduced extent to prepare a virion strand, i.e. the genomic virus RNA or DNA.

A preferred embodiment of the present invention is an RNA molecule which additionally carries a poly-(A) sequence. The term "poly(A) sequence" means that onto the 3' end of the primary transcript a poly(A) homopolymer is synthesized after transcription. On the level of the primary transcript shortly before the 3' end of said transcript a signal sequence of, e.g., 6 nucleotides must be present. Such a poly(A) sequence is decisive for the stability of the RNA and for its transport from the cell nucleus to the cytoplasm.

A further preferred embodiment of the present invention is an RNA molecule which is an antisense RNA. The term antisense RNA relates to an RNA which is substantially complementary to the binding site of the substrate RNA and which may exhibit some further vector-specific bases both at the 5' and at the 3' end and which may exhibit a poly(A) sequence at the 3' end.

In a particularly preferred embodiment the antisense RNA according to the present invention binds to an RNA sequence of the viral RNA replicative intermediate, with the RNA sequence of the viral RNA replicative intermediate either corresponding to an open reading frame or being complementary to an open reading frame.

A further preferred embodiment of the present invention is an RNA molecule exhibiting a ribozyme activity. In a particularly preferred embodiment said RNA molecule binds to a sequence in the viral RNA replicative intermediate which contains the sequence motif "GUX". The term "ribozyme activity" means that a substrate RNA molecule is cleaved in a specific site, namely at the 3' end facing the nucleotide X of the GUX consensus sequence so that said substrate RNA molecule after cleavage is present in the form of two linear molecules. The nucleotide X of the consensus sequence refers to nucleotides with the bases C, U or A.

A further preferred embodiment of the present invention is an RNA molecule which binds to a viral RNA replicative intermediate of an animal or plant RNA or DNA virus.

Examples of such animal viruses are:
( + ) RNA viruses
   Picornaviridae, e.g. polioviruses (1 RNA chromosome)
   Caliciviridae, e.g. feline calicivirus (1 RNA chromosome)
   Togaviridae , e.g. Sindbis virus (1 RNA chromosome)
   Flaviviridae , e.g. hepatitis C (1 RNA chromosome)
(-) RNA viruses
   Rhabdoviridae , e.g. vesicular stomatitis virus (1 RNA chromosome)
   Orthomyxoviridae, e.g. influenza virus (8 RNA chromosomes)
   Paramyxoviridae, e.g. Sendai virus (1 RNA chromosome)
   Arenaviridae, e.g. Lassa fever virus (2 RNA chromosomes)
   Bunyaviridae , e.g. La Crosse virus (3 RNA chromosomes)
DNA viruses
   Hepatitis B virus
   Examples of plant RNA viruses are:
( + ) RNA viruses
   Tobamoviridae, e.g. tobacco mosaic virus (1 RNA chromosome)
   Comoviridae, e.g. cowpea mosaic virus (2 RNA chromosomes)
   Tobraviridae, e.g. tobacco rattle virus (2 RNA chromosomes)
   Cucumoviridae, e.g. cucumber mosaic virus (3 RNA chromosomes), alfalfa mosaic virus (3 RNA chromosomes)
   Bromoviridae, e.g. bromo mosaic virus (3 RNA chromosomes)
(-) RNA viruses
   Rhabdoviridae, e.g. lettuce necrotic yellow virus (1 RNA chromosome)
   Bunyaviridae, e.g. tomato spotted wilt virus (3 RNA chromosomes, with the smallest RNA exhibiting an ambisense coding pattern).

In a particularly preferred embodiment the RNA molecule of the present invention binds to a replicative intermediate derived from the RNA virus TSWV (tomato spotted wilt virus). TSWV is a representative of the genus tosbo virus (de Haan et al., 1989, 1990) which belongs to the family of bunyaviridae. It has a very wide range of hosts. Presently 370 species in 50 families are described (R. Kormelink et al., 1991) which are infested with said virus. It is transferred via thrips (thripidae) and both dicotyledonous and monocotyledonous plants are infested. TSWV is a (-) strand virus whose genome consists of three single-stranded RNA molecules which are different in size (SRNA: about 2.9 kb; MRNA: about 5.2 kb; LRNA: 8.9 kb). In the LRNA and the MRNA the (-) strand is packaged in the virions, i.e. only the complementary strand synthesized in the plant carries the correct open reading frame (ORF) and can be translated in the cell. The LRNA codes for the viral replicase (de Haan et al., 1991) and the MRNA (at least) for two glycoproteins (Elliott et al., 1991). The SRNA has a so-called ambisense coding strategy. One ORF corresponds to the RNA molecule present in the virions and a second ORF is represented by the complementary RNA molecule. Both ORFs do not overlap but are separated by a so-called intergenic region. Both proteins coded for by the SRNA are translated by subgenomic RNAs. The smaller protein (28.9 kDa) is a nucleoprotein (de Haan et al., 1990) which binds to the genomic nucleic acid of the virus. The bigger protein (52.4 kDa) has recently been detected in the cytoplasm (Kormelink et al., 1991).

In a further particularly preferred embodiment the RNA molecule of the present invention contains one of the RNA sequences depicted in Figures 1 to 4 which are complementary to certain parts of the TSWV replicative intermediate.

Figure 1 shows part of the TSWV substrate RNA molecule and the complementary and preserved region of the ribozyme specific of the first selected TSWV-GUC site (replicative intermediate). The RNA molecule of the present invention is that designated as ribozyme RNA. In the following said ribozyme is referred to as RZ2.
Figure 2 shows part of the TSWV substrate RNA molecule and the complementary and preserved region of the ribozyme specific of the second selected TSWV-GUC site (replicative intermediate). The RNA molecule of the present invention is that designated as ribozyme RNA. In the following said ribozyme is referred to as RZ3.
Figure 3 shows an antisense RNA molecule (AS3) according to the invention. It is complementary to the replicative intermediate in the coding region of the NS₅ protein.
Figure 4 shows an antisense RNA molecule (AS4) according to the invention. It is complementary to the replicative intermediate in the coding region of the N protein.

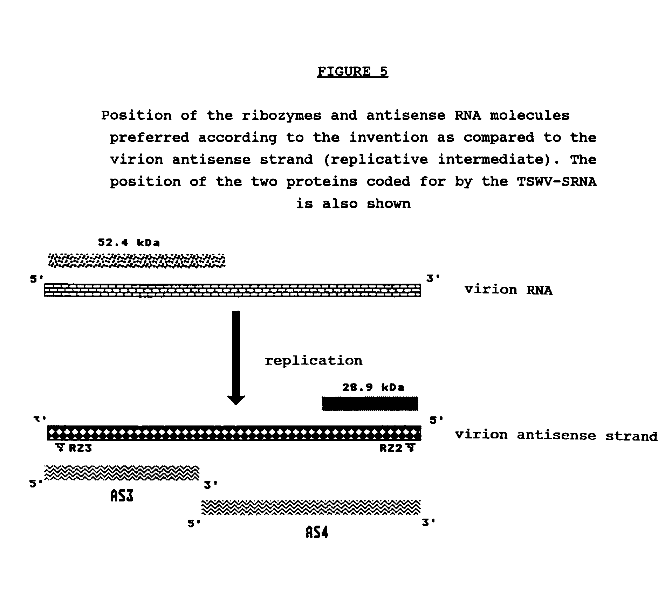

Figure 5 explains to which sites of the TSWV replicative intermediate the above mentioned ribozymes RZ2 and RZ3 and the above mentioned antisense RNA molecules AS3 and AS4 are complementary.
Figure 6 shows part of the HBV substrate RNA molecule and the complementary region of the ribozyme specific of the selected HBV-GUC site (replicative intermediate). The RNA molecule according to the invention is that designated as ribozyme RNA. In the following said ribozyme is referred to as RZ6.
Figure 7 shows part of the HCV substrate RNA molecule and the complementary region of the ribozyme specific of the selected HCV-GUC site (replicative intermediate). The RNA molecule according to the invention is that designated as ribozyme RNA. In the following said ribozyme is referred to as RZ7.

The RNA molecules according to the invention can be prepared synthetically and/or by genetic engineering techniques.

A further subject matter of the invention is a DNA sequence which codes for or transcribes one of the above RNA molecules according to the invention.

A further subject matter of the invention is a gene having the following features:
(a) it exhibits a promoter being suitable to control transcription in a desired host cell, and
(b) it contains one of the above-mentioned DNA sequences according to the present invention.

In a preferred embodiment of the present invention the gene, in addition to the above features, exhibits at least one of the following features:
(c) a transcription termination sequence (TTS), and
(d) a polyadenylation site.

The term "transcription termination sequence" means that it refers to a sequence which defines the end of the RNA synthesis of a certain DNA sequence serving as matrix by a certain polymerase. In the case of the "chimeric genes" used in transgenic plants, e.g. the TTS of the nopaline synthetase gene (Zambryski et al., 1983), of the octopine synthetase gene (Herrera-Estrella et al., 1983) or of the 35S transcript of CaMV (cauliflower mosaic virus) (Pietrzak et al., 1986) can be used.

The term "polyadenylation site" preferably refers to a sequence consisting of six nucleotides (e.g. AAUAAA) which effects the exact processing of the primary transcript and the posttranscriptional synthesis of a polyA sequence to the 3' end of the primary transcript.

In a preferred embodiment of the present invention the gene is a chimeric gene. The term "chimeric gene" means that it refers to a composite gene in which at least one of the respective control regions and signal sequences, e.g. above features (a) to (d), does not have the same origin as the gene to be transcribed.

A further subject matter of the invention is a vector containing one of the above DNA sequences of the invention or one of the above genes or chimeric genes of the invention. The term "vector" refers to a linear or circular nucleic acid sequence which can be present as single strand or double strand either as "naked" nucleic acid (e.g. plasmid) or "packaged" nucleic acid (e.g. X phage).

A vector contains specific sequences which are necessary for replication and selection. In addition to these sequences a recombinant vector contains the desired DNA sequence to be incorporated into a cell. For example, as plant vectors derivatives such as pMEX001 of a binary vector system were used (Koncz and Schell, 1986). It is referred to as a binary vector which is suitable for the transformation of agrobacteria but which also replicates in E. coli.

A further subject matter of the present invention is a host organism containing the vector of the present invention exhibiting the above-mentioned features.

In a preferred embodiment the host organism is a microorganism, preferably of the genus Escherichia or Agrobacterium, more preferably of the species Escherichia coli or Agrobacterium tumefaciens, a plant or animal cell.

A further subject matter of the invention is a host organism carrying in its genome one of the above-mentioned DNA sequences according to the invention or one of the above-mentioned genes or chimeric genes according to the invention. Preferred are microorganisms, plant and animal cells. Via expression of the DNA sequence or the gene or chimeric gene the respective RNA sequence is produced which inhibits the viral growth cycle of the RNA or DNA virus due to its stable bond to the corresponding viral RNA replicative intermediate. A sufficiently strong inhibition of the viral growth cycle leads to an increase in the virus resistance of the host organism.

A further subject matter of the present invention is a virus-resistant transgenic plant which has been regenerated from a plant cell according to the present invention and which contains in its genome one of the above DNA sequences, genes or chimeric genes according to the invention. The invention also relates to the progeny of these plants. In this context the term "regeneration" means growing transformed plant cells produced by, e.g. leaf cutting transformation, by coculturing regenerating plant protoplast cell cultures with Agrobacterium tumefaciens or by direct DNA transfection in suitable culture media to complete transgenic plants. The transformed plant cells are cultivated and regenerated to complete plants according to conventional methods with the help of appropriate culture media (Nagy and Maliga, 1976). Transgenic plants obtained in this way can be identified via the expression of a reporter gene (marker gene), if necessary.

For the transfer of foreign DNA into genomes of dicotyledonous and monocotyledonous plants the Ti plasmid of Agrobacterium tumefaciens is available as a particularly useful and widely applicable vector. The DNA sequence according to the invention or the chimeric gene according to the invention is inserted between the border sequences or in the region of the right border sequence of appropriate Ti plasmids (Zambryski et al., 1983) and is transferred by infection of the plant, infection of plant parts or plant tissues such as leaf cuttings, stems, hypocotyls, cotyledons, meristems and tissues deriving thereof such as secondary embryos and calli, or by coculture of protoplasts with Agrobacterium tumefaciens. Transformation of the cells can be also achieved by direct DNA introduction. Said introduction can be favourably influenced by an electric field (electroporation), if necessary (Fromm et al., 1986).

A further subject matter of the invention is the propagation material of the transgenic plants of the present invention.

In this context the term "plant" refers to both complete plants and plant parts such as leaves, seeds, bulbs, cuttings, etc. The term "propagation material" refers to plant parts and plant cells that can be used for the propagation of transformed plants. Examples of such plant parts are seeds or cuttings.

A further subject matter of the invention is a kit for the preparation of virus-resistant transgenic host organisms containing one or more of the above DNA sequences according to the invention, one or more of the genes or chimeric genes according to the invention, or one or more of the vectors according to the invention, or a combination thereof.

A preferred embodiment is a kit which can be used for the preparation of a virus-resistant plant cell, of a virus-resistant plant or a virus-resistant animal cell. The term "plant cell" includes protoplasts, cell lines and plant calli.

The virus-resistant animal cells according to the invention can for instance be used for somatic cell therapy.

Finally, the subject matter of the invention is an agent for treating viral infections comprising at least one RNA molecule, DNA molecule, gene or chimeric gene according to the invention, or at least one vector according to the invention, or a combination thereof.

The examples serve to illustrate the invention.

### Example 1

### 1. Construction of vectors

### 1.1 RZ2 and RZ3 (construction of ribozymes)

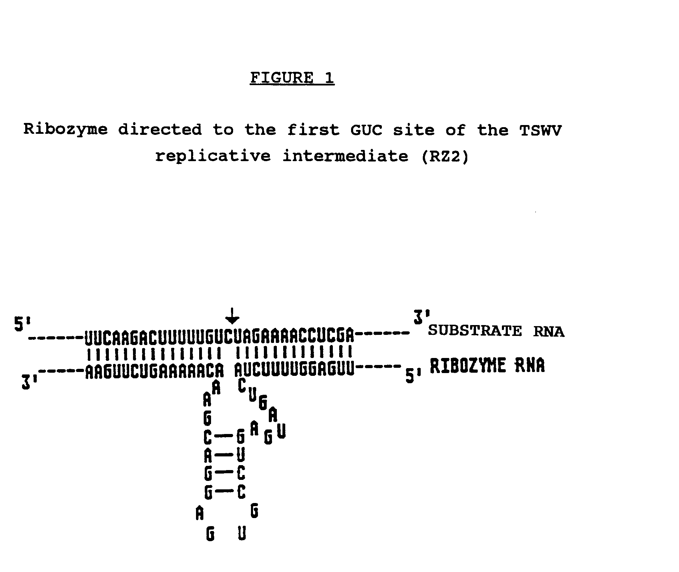

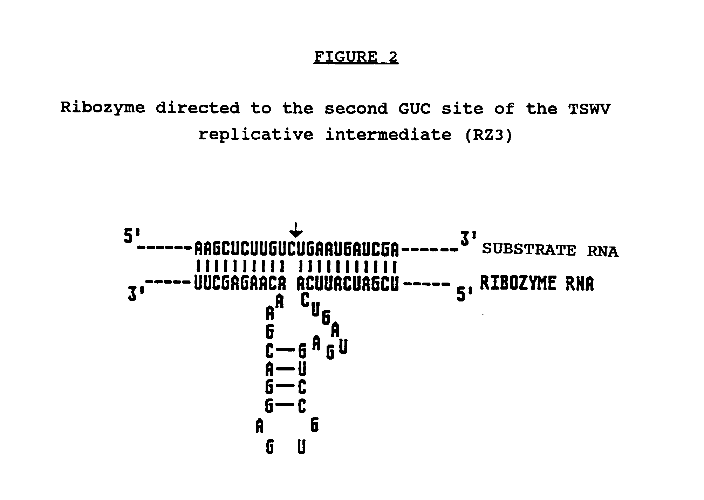

Complementary oligonucleotides coding for the desired ribozyme RZ2 or RZ3 (Fig. 1 or 2) were prepared with a DNA synthesis device (380B of Applied Biosystems) and phosphorylated at the 5' end using polynucleotidekinase. Since the oligonucleotides were designed so as to provide corresponding protruding 5' sequences after hybridization, the base-paired oligonucleotides could be cloned into a pBLUESCRIPT KS plasmid (Stratagene) which was digested with the restriction endonucleases EcoRl and Hindlll. The reaction products served to transform E.coli DH5a cells. The DNA of positive recombinants was digested with the restriction endonucleases Hincll and BamHl and the insertion fragment was cloned into a pMEX001 vector which was digested with Smal/BamHl. The plasmid pMEX001 used carries one gene for the ampicillin resistance and one gene for the methotrexate resistance and is 8,650 bp in size. It can be conventionally propagated in Escherichia coli cells. It is a binary vector which can replicate not only in Escherichia coli cells but also in Agrobacterium tumefaciens cells. The plasmid includes a 35S promoter, a polylinker and a 35S termination sequence as well as a polyadenylation signal.

The thus constructed RZ2 and RZ3 containing plasmids pRZ2 and pRZ3, respectively, were transferred to Agrobacterium tumefaciens GV3101 (Koncz and Schell, 1986) by direct transformation. The resulting Agrobacterium strains were then used for plant transformation.

### 1.2 AS3 and AS4 (antisense constructions)

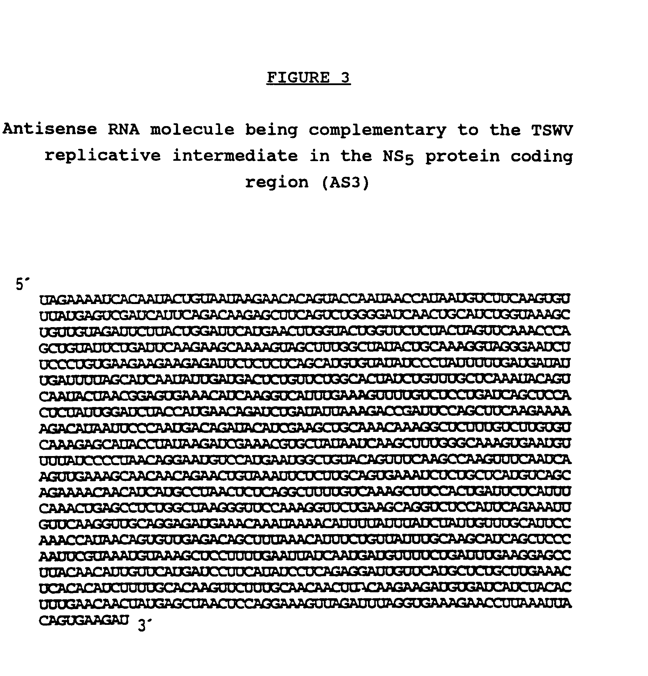

1.2.1 As3 (Fig. 3) BamH̅l̅/̅E̅c̅oRV digestion of the clone TSWV-L3/335 (Maiss et al., 1991) afforded a fragment of 1.18 kbp in size which was cloned into a pMEX001 vector digested with Xbal (filled in)/BamHl behind the 35S promoter. The ligation preparation served to transform competent E.coli DH5a cells. The DNA of a positive recombinant was used to transform competent Agrobacterium tumefaciens cells of the GV3101 strain (Koncz and Schell, 1986). The resulting Agrobacterium strain was then used for plant transformation.

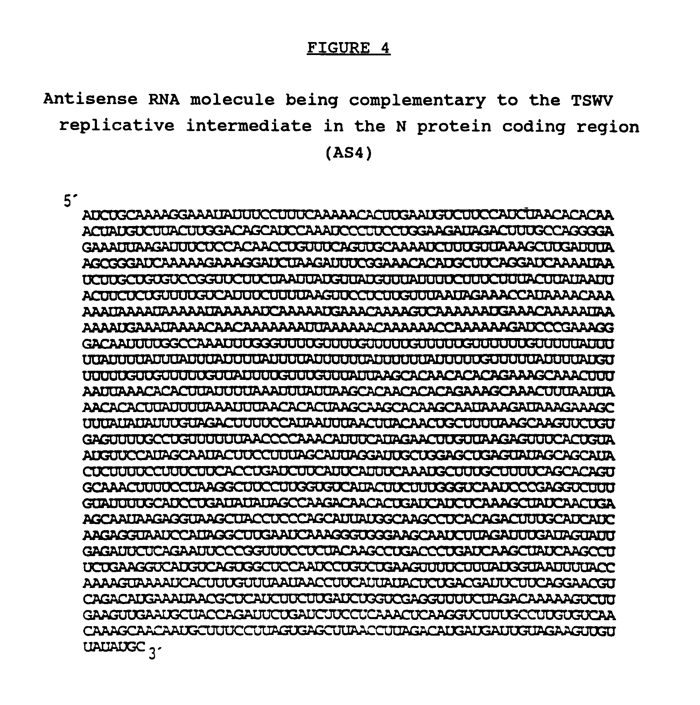

1.2.2 As4 (Fig. 4) BamH̅I̅/̅E̅c̅oRV digestion of the clone TSWV-L3/308 (Maiss et al., 1991) afforded a fragment of 1.7 kbp in size which was cloned into a pMEX001 vector digested with Smal/BamHl behind the 35S promoter. The ligation preparation served to transform competent E.coli DH5a cells. The DNA of a positive recombinant was used to transform competent Agrobacterium tumefaciens cells of the GV3101 strain (Koncz and Schell, 1986). The resulting Agrobacterium strain was then used for plant transformation. 1.2.3 The Escherichia coli strain DH5a which contains the vector MOMPI335B (coded for AS3) or MOMPI308A (coded for AS4) in easily isolatable form was deposited with Deutsche Sammlung von Mikroorganismen (DSM), Griesebachstr. 8, W-3400 Göttingen, Federal Republic of Germany, in accordance with the regulations of the Budapest Treaty and has deposit nos. DSM 6735 (MOMPI335B) and DSM 6734 (MOMPI308A).

### 2. Transformation of plants

### 2.1 Transformation of Agrobacterium

2.1.1 For the transformation of tobacco plants leaf cuttings (Horsch et al., 1985) were used. Leaves of sterile shoot cultures of 2-3 cm in length were cut out in slices of 1 cm diameter and incubated at 26 _{°} C for 60 hrs with an Agrobacterium culture which was grown for 24 hrs and added to 10 mM MgS0₄ and 20 ml MS medium (Murashige and Skoog, 1965). The leaf cuttings were then washed 3 times in MS medium with 1,000 µg Claforan and spread on MS plates which contained 0.5 µg/ml methotrexate, 0.5 µg/ml NAA (naphthyl acetic acid), 0.2 mg/ml BAP (benzylamine purine) and 2 % sucrose ("callus induction medium"), and incubated at 26 _{°} C. After every two weeks the leaf cuttings were transferred to a new callus inducing medium. As soon as small calli could be detected they were transferred to a shoot induction medium which corresponds to the callus induction medium but contains 0.2 µg/ml NAA and 0.5 µg/ml BAP. Once the regenerated shoots were 2-3 cm in length, they were transferred to MS medium containing 1 % sucrose but no hormones for root induction and cultivated under sterile conditions at 24 - 26°C (12 hrs light (1,000 - 3,000 lux), 12 hrs darkness). After 4 to 6 weeks the shoot cultures were transferred to fresh medium, grown to plants and then cultivated under appropriate conditions in the greenhouse.

2.1.2 For transformation of potato plants the leaf cuttings were incubated in the Agrobacterium suspension (2.1.1) and then incubated for further two days in darkness. The leaf cuttings were then incubated for 8 days at 16 hrs exposure to light on MS plates containing 1.6 % glucose, 5 mg/I NAA, 0.1 mg/I BAP, 500 mg/I Claforan and the respective selection marker. The leaf cuttings were transferred to MS plates containing 1.6 % glucose, 2.0 mg/I zeatine riboside, 0.02 mg/I NAA, 0.02 mg/I GA3 (giberrilinic acid) and 500 mg/I Claforan. After 14 days of incubation the leaf cuttings were transferred to fresh plates. First calli appeared after about 6 to 7 weeks. Appearing shoots were transferred to 250 ml glass tubes containing MS medium with 3 % sucrose and 0.5 mg/ml carbenicillin. First roots were observed after about 14 days. The plants were then cultivated under appropriate conditions in the greenhouse.

2.1.3 Transformation of tomato plants substantially proceeded according to the method described in item 2.1.2.

2.1.4 From the plants obtained DNA (Dellaporta et al., 1983) and RNA (Goodall et al., 1990; Chomczynski and Sacchi, 1987) were isolated using standard techniques. DNA analysis (southern blot) served to determine the integrity and quantity of transferred genes, RNA analysis (RNase protection according to Vankan and Filipowicz, 1988; Goodall and Filipowicz, 1989; Steinecke et al., 1992) served to determine the transcript amount (cf. item 3).

### 2.2 Protoplast isolation and PEG mediated transformation

4-8 week old Nicotiana tabacum SR1 leaves from a sterile culture were cut off, cleaned with H₂0 dist. and laid in a 0.05 % SDS solution to avoid bacterial contamination. The SDS was removed by twice washing with sterile H₂0. The leaves were equilibrated for 10 min in K3 medium (500 mg/I Claforan, 1 mg/I NAA, 0.2 mg/I kinetine). The mid-rib was removed and the leaves were cut into 1-2 cm² pieces. For isolation of the mesophyll protoplasts the leave pieces were put in a sterile 1 I bottle with 30 ml of an enzyme solution containing cellulase and mazerozyme (1.5 % cellulase, 0.5 % mazerozyme, in K3 medium) and subjected to a mild vacuum for 10 min. After 16 hrs incubation at 27 _{°} C in darkness the preparation was agitated for 30 min at 75 rpm. The protoplast suspension was passed through steel sieves having 250 and 100 µm pore size, the filtrate was distributed in 12 ml centrifuge tubes and centrifuged for 5 min at 650 rpm in a Hettich centrifuge Universal 2S. The bottom phase containing the precipitate was drained off with a 100 µm glass capillary tube, the protoplasts were washed with 10 ml K3 medium and the medium was drained off after a further centrifugation (cf. above). Then the protoplasts were added to W5 medium (154 mM NaCI, 125 mM CaC1₂xH₂0, 5 mM glucose, 5 mM KCI, pH 5.6) and counted in a Fuchs-Rosenthal counter and left to stand for 30 min in W5 medium. The protoplasts were separated by 5 min centrifugation at 650 rpm and the precipitate was suspended in a MaMg solution (450 mM mannite, 15 mM MgC1₂, 0.1 % MES, pH 5.6) with the density being adjusted at 1-3x10⁶ protoplasts/ml. The protoplasts could then be transfected either immediately or could be kept in a refrigerator up to 4 hrs.

For transformation the protoplasts were subjected to a 5 min heat shock at 45 _{°} C in a hot water bath under occasional agitation and then immediately cooled down to room temperature for 45 s in ice/water. Then portions of 0.35 ml protoplast suspensions were distributed to 10 ml centrifuge tubes and the DNA solution was added in a volume of 50 µl. After 10 min 0.35 ml PEG solution (100 mM Ca(N0₃)₂x4H₂0, 400 mM mannite, 40 % PEG 4000, pH 7-9) were added dropwise. After a further 20 min, during which the preparations were agitated every 5 min, the transfection preparation was transferred to petri dishes. 4 ml K3 medium was added dropwise and the protoplasts were cultivated at 27°C for 6-48 hrs in the dark. After incubation the protoplasts were put in 12 ml centrifuge tubes which were filled with sea water for washing, and centrifuged for 3 min at 650 rpm. The supernatant was removed except for 1 ml and the protoplasts were resuspended and transferred to Eppendorf vials. After centrifugation for 1 min at 13,000 rpm (Biofuge) and carefully removing the supernatant the protoplasts were added to the respective extraction buffers.

In protoplasts prepared according to the above described method e.g. TSWV viruses or parts thereof could be incorporated. In correspondingly treated protoplasts the replication of viral RNA and the effectiveness of inhibition of the RNA molecules according to the invention which are incorporated into the protoplasts by e.g. vectors can be proven. The results show that already at this early stage an inhibition of viral replication could be observed (cf. also item 3).

### 3. RNA analysis

### 3.1 RNA extraction from protoplasts (Steinecke et al.. 1992)

An inhibition of the viral replication or the cleavage of viral RNA by the inventive transcribed RNA molecules of the transformed protoplasts was proven with the help of the method described below. This method (according to Goodall et al., 1990; Chomczynski and Sacchi, 1987) provides an RNA yield of 5-25 µg RNA from 6x10⁵ protoplasts. After centrifugation the protoplasts of a single transformation were lysed in an Eppendorf vial with 0.4 ml solution D (4 M guanidine thiocyanate, 25 mM sodium citrate (pH 7.0), 0.5 % sarkosyl, 0.1 M 2-mercaptoethanol) by vigorous mixing. 40 µl 2 M sodium acetate (pH 4.0), 400 µl phenol and 80 µl chloroform/isoamyl alcohol were added, the entire preparation was mixed well and incubated for 15 min on ice. It was centrifuged for 20 min at 4 _{°} C and 13,000 rpm (Biofuge), the aqueous supernatant was transferred to a new Eppendorf vial with 400 µl isopropanol and the RNA was precipitated for 15 min at 20 °C. After 15 min centrifugation at 4°C and 13,000 rpm (Biofuge) the precipitate was dried shortly and then added to 0.3 ml solution D, mixed with an equal volume of isopropanol and precipitated for 15 min at -20 °C. After centrifugation the precipitate was washed with 200 ml 75 % ethanol, dried shortly in the air and dissolved in 50 ml DEPC-treated H₂0 with 6 µl deionized formamide.

To the dissolved RNA 6 ml 10xDNase buffer (100 mM Tris-HCI (pH 8.0), 100 mM MgC1₂), 1 µl RNasine (10 U/µl) and 1 µl RNase-free DNase (1 U/µl) were added. After 30 min incubation at 37°C 100 µl TE buffer and 160 µl phenol/chloroform/isoamyl alcohol were added, mixed well and centrifuged for 5 min at 13,000 rpm (room temperature)(Biofuge). The aqueous phase was transferred to a new Eppendorf vial and the RNA was precipitated with ethanol. The precipitate was dissolved in 50 ml DEPC-treated H₂0.

### 3.2 RNase protection test

The RNase protection test (Vankan and Filipowicz, 1988; Goodall and Filipowicz; Steinecke et al.) constitutes a very sensitive method for the detection and the quantification of RNA species in cellular whole RNA. A radioactively labelled RNA probe is synthesized which is mostly complementary to the target RNA to be analyzed.

For every preparation 2x10^{4 -} 1×10⁵ cpm ³²p or ³⁵ S labelled antisense probe with 1-5 µg whole RNA were precipitated in an Eppendorf vial for 15 min at -70 °C in the presence of 300 mM sodium acetate with 2.5 vol ethanol. Then the preparation was centrifuged for 15 min in a Sorvall SS34 rotor having Eppendorf inserts at 11,000 rpm and 4 _{°} C. After carefully removing the supernatant every precipitate was added in 20 µl solution A (80 % deionized formamide, 40 mM PIPES (pH 6.4), 400 mM sodium acetate (pH 6.4), 1 mM EDTA) by shortly agitating and centrifuging. The reaction mixture was denatured for 4 min at 90 °C, shortly centrifuged and incubated at 45-48 °C over night in a heating block.

After hybridization it was shortly centrifuged, 200 µl of a 1/50 dilution of the RNase restriction solution R (50 U/ml RNase A, 10,000 U/ml RNase Tᵢ) and 50 U/ml RNase T₂ were added and the RNase treatment was carried out according to the method of Vankan. After shortly agitating and centrifuging (Biofuge) the preparations were incubated for 40 min at 37 _{°} C. Solution D2 (20 % SDS) was mixed with an equal volume of solution D1 (10 mg/ml proteinase K), 20 ml of each were added to the preparations and were further incubated for 15 min at 37 °C. The mixture was put into a new Eppendorf vial with 250 µl phenol/chloroform/isoamyl alcohol. After thoroughly mixing it was centrifuged for 5 min and the supernatant was transferred to a new Eppendorf vial with 625 µl 96 % ethanol. The reaction mixture was shortly agitated, precipitated for 15 min at -70 °C and centrifuged for 15 min at 4 °C in a Sorvall SS34 rotor having Eppendorf inserts at 11,000 rpm. The supernatant was carefully removed, the precipitate was dried for 15 min and added to 8 ml solution E (80 % formamide, 0.1 % xylene cyanol, 0.1 % bromophenol blue, 2 mM EDTA). The preparations were denatured for 4 min at 90 _{°} C, centrifuged and separated for 2 hrs at 20 mA on a 5-9 % polyacrylamide gel. The gel was transferred to 3MM paper, covered with saran foil and dried. Autoradiography was carried out with an intensifying screen (Rapidscreen, Kodak) at -70 °C.

RNA analysis showed that the amount of viral RNA in protoplasts cotransfected with the RNA molecules according to the invention was considerably lower than that in mock-infected protoplasts.

### 4. Method for testing transgenic plants for TSWV resistance

### 4.1 Propagation of the plants

The seeds of transgenic plants were sown in FloraDur (type B) culture substrate (Floragard GmbH) and placed at 20-25 _{°} C under greenhouse conditions. After appearance of the cotyledons (about 2 weeks) the embryos were transplanted into transplant trays with FloraDur (type B) culture substrate and cultivated for 3-4 weeks. The young plants were then planted into pots of 10 cm diameter and were fertilized twice a week with a 0.2 % Wuxal solution (Aglukon) (8/8/6).

### 4.2 Inoculation of the plants

From tobacco plants (Nicotiana rustica L.) which 3 weeks previously were infected with the TSWV isolate L3 (DSM No. PV0182), 3 g leaf material were harvested from primarily infected leaves showing clear virus symptoms and were homogenized in 30 ml standard buffer (0.1 M Na-K phosphate buffer pH 7.0 with 0.2 % polyvinylpyrrolidone MW 10000 and 0.2 % (w/v) Na₂SO₃) in a mortar. A 1:25 dilution thereof was prepared in the standard buffer and 0.1 ml per leaf onto 3 leaves each of 8-10 week test plants was applied mechanically by rubbing the leaves with a cotton tip. The test plants were previously sprayed with an abrasive (Carborundum 600 mesh) and rinsed with tap water after inoculation.

### 4.3 Appraisal of the plants, ELISA and evaluation

The development of the symptoms on the plants was visually evaluated with an appraisal scheme as of the 5th day after inoculation over a period of 14 days.
Stage 0 = without symptoms
Stage 1 = mild symptoms, circular spots on the leaves (necroses)
Stage 2 = strong symptoms, yellowing nervature, mosaic necrotic symptoms
From the appraisal values the average value was determined and correlated with the values of the control plants.

For a further quantification of the resistance of the plants a triple antibody sandwich (TAS) ELISA process was used to determine the TSWV antigen amount (Casper and Meyer, 1981). Greiner microtiter plates (type 655001) were coated with 0.1 ml polyclonal antiserum (DSM No. AS-0105, 2 µg/l) per well. Pressed juices of test plants were prepared from primarily and secondarily infected leaves in dilutions of 1:30 and 1:500 with the standard ELISA buffer. 3 wells each of the plate were filled with every sample (0.1 ml) and incubated over night at 4 _{°} C. After three washing steps with PBS Tween 0.1 ml per well of a 1:1,000 dilution of the two monoclonal antibodies 4F2 and 2B6 (Adam et al., 1991) was added and incubated for 3 hrs at 37 _{°} C. After three washing steps with PBS Tween 0.1 ml per well of a 1:1,000 dilution of a rabbit anti-mouse IgG conjugate (DAKOPATTS, No. D314) was added and incubated for 3 hrs at 37 _{°} C. After three washing steps with PBS Tween the substrate p-nitrophenyl phosphate (1 mg/ml) was added and the extinction was measured at 405 nm in a photometer after 30 min and 1 h. The average extinction value was determined from the extinction values and correlated to the control values. The results show that the transgenic plants show either none or only mild symptoms while the control plants show strong symptoms. Moreover, it became evident that the TSWV antigen quantity in the transgenic plants is considerably lower than that in the control plants.

### 5. Inhibition of the HBV or HCV replication in animal cells using RNA molecule RZ6 or RZ7

### 5.1 Transfection of HBV or HCV infected animal cells with vector pCDNAlneo containing RZ6 or RZ7

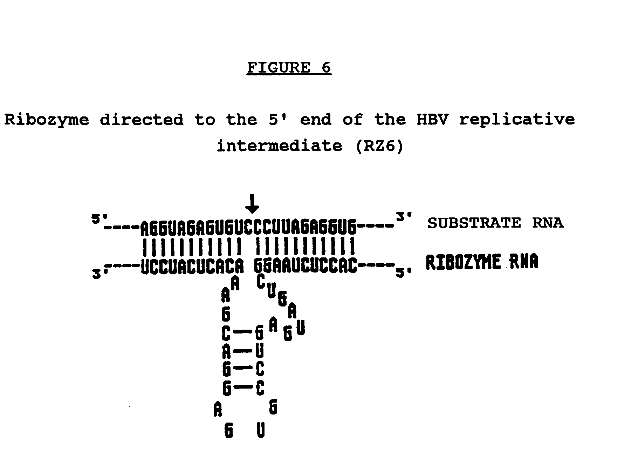

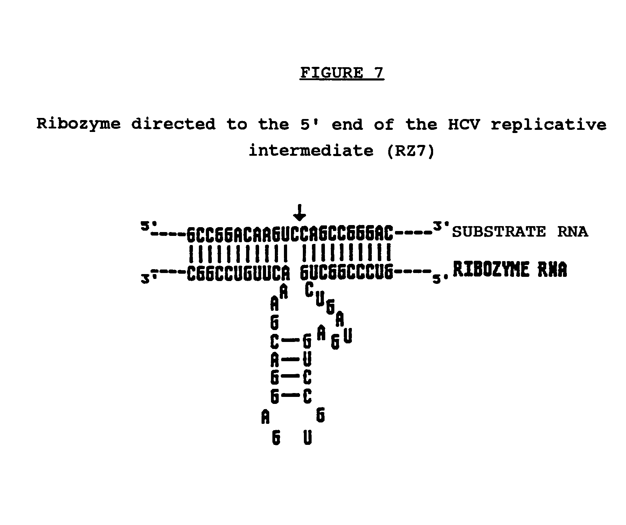

Complementary oligonucleotides coding for the desired ribozyme RZ6 or RZ7 (Fig. 6 or Fig. 7) were prepared as described in item 1.1 and functionally cloned into vector pCDNAlneo (company in vitro-Gen). The vector construct pRZ6 containing RZ6 was used to transfect the hepatoma cell line HepG2.215 (Sells et al., 1987). This cell line expressed the entire HBV-genome.

The vector construct pRZ7 containing RZ7 was used to transfect monocytes. These monocytes expressed the HCV genome when appropriately stimulated.

### 5.2 Assay of the inhibition of the viral replication

The hepatoma cells transfected with pRZ6 were assayed for their HBV-RNA and HBV DNA content and analyzed for the HBV replicative intermediates using hybridization methods well known in the art and the RNase protection test described in item 3.2. Furthermore, the quantity of HBV antigens was determined in an ELISA test (Behring). The monocytes transfected with pRZ7 were also assayed for their HCV-RNA content and analyzed for their HCV replicative intermediates as described above.

The results showed that both in the pRZ6 transfected hepatoma cells and the pRZ7 transfected monocytes a considerable inhibition of the HBV or HCV replication as compared to the corresponding control cells can be observed.

These results were confirmed for the hepatoma cells transfected with pRZ7 in a further examination of the culture supernatant for HBV particles ("Dane particles") by electron microscopy.

### References

Abrahams et al., Nucleic Acids Res. 18 (1990), 3035-3044 Adam et al., Annals of Applied Biology (1991), 87-104 Beachy, "Plant transformation to confer resistance against virus infection" in: "Gene manipulation in plant improvement II", Plenum Press, New York, 1990 Chomzynski and Sacchi, Anal. Bioch. 162 (1987), 156-159 Dellaporta et al. Plant Mol. Biol. Rep. 1 (1983), 19-21 Cotten and Birnstiel, EMBO J. 8 (1989), 3861-3866 Elliott et al., "Bunyaviridae genome structure and gene expression" in: "Current Topics in Microbiology and Immunology", Vol. 169, 91-141, Springer Verlag, Berlin Heidelberg 1991 Fromm et al., Nature 319 (1986), 791-793 de Haan et al., J. Gen. Virology 70 (1989), 3469-3473 Goodall et al., Meth. in Enzym. 181 (1990), 148-161 Goodall and Filipowicz, Cell 58 (1989), 473-483 de Haan et al., J. Gen. Virology 7 (1990), 1001-1007 Hemenway et al., EMBO J. 7 (1988), 1273-1280 Herrera-Estrella et al., Nature 303 (1983), 209-213 Izant and Weintraub, Cell 36 (1984), 1007-1015 Koncz and Schell, Mol. Gen. Genet. 204 (1986), 338-396 Kormelink et al., Virology 181 (1991), 459-468 van der Krol et al., Biotechniques 6 (1988), 958-976 Maiss et al., J. Gen. Virol. 72 (1991), 461-464 Nagy and Maliga, Z. Pflanzenphysiol. 78 (1975), 453-455 Nassuth and Bol, Virology 124 (1983), 75-85 Palukaitis and Zaitlin, "A model to explain the "Cross-Protection" phenomenon shown by plant viruses and viroids", in: "Plant Microbe Interactions, Molecular and Genetic Perspectives", Vol. 1, Kosuge and Nestor, eds. Maxmillan, New York, 1984, 421-429 Pietrzak et al., Nucleic Acid Res. 14 (1986), 5857-5868 Powell et al., Proc. Natl. Acad. Sci. USA 86 (1989), 6949-6952 Rezaian et al., Plant Mol. Biol. 11 (1988), 463-471 Sells et al., Proc. Natl. Acad. Sci. USA 84 (1987), 4641-4646 Steinecke et al., EMBO J. 11 (1992), 1525-1530 Symons (1989), TIBS 14 (1989), 445-450 Vankan et al., Nucleic Acid Res. 16 (1988), 10415-10430 Zambryski et al., EMBO J. 2 (1983), 2143-2150.

## Claims

1. An RNA molecule having the following features:
(a) it exhibits a sequence which is complementary to at least one part of a viral RNA replicative intermediate;
(b) it can intracellularly form a stable bond with the viral RNA replicative intermediate;
(c) it binds to a part of the viral RNA replicative intermediate which is available under intracellular conditions for such a bond; and
(d) the stable bond to the viral RNA replicative intermediate inhibits the viral growth cycle.

2. The RNA molecule according to claim 1 which additionally carries a poly(A) sequence.

3. The RNA molecule according to any of claims 1 and 2 which is an antisense RNA.

4. The RNA molecule according to any of claims 1 to 3 wherein the antisense RNA binds to an open reading frame of the viral RNA replicative intermediate.

5. The RNA molecule according to any of claims 1 to 3 wherein the antisense RNA binds to an RNA sequence of the viral RNA replicative intermediate said RNA sequence being complementary to an open reading frame.

6. The RNA molecule according to any of claims 1 to 5 which exhibits ribozyme activity.

7. The RNA molecule according to claim 6 which binds to a sequence in the viral RNA replicative intermediate containing the sequence motif "GUX", with X meaning a nucleotide having the base C, U or A.

8. The RNA molecule according to any of claims 1 to 7, wherein the viral RNA replicative intermediate is derived from an animal or plant RNA or DNA virus.

9. The RNA molecule according to claim 8, wherein the plant RNA virus is TSWV (tomato spotted wilt virus).

10. The RNA molecule according to claim 9 which contains one of the TSWV ribozyme sequences depicted in Figures 1 and 2, a TSWV-NSs antisense sequence according to Figure 3 or a TSWV-N antisense sequence according to Figure 4.

11. A DNA sequence which codes for an RNA molecule according to any of claims 1 to 10.

12. A gene having the following features:
(a) it exhibits a promoter which is suitable in a desired host cell to control transcription; and
(b) it contains a DNA sequence according to claim 11.

13. The gene according to claim 12 which additionally exhibits at least one of the following features:
(c) a transcription termination sequence; and
(d) a polyadenylation site.

14. The gene according to claim 12 or 13 which is a chimeric gene.

15. A vector which contains a DNA sequence according to claim 11, a gene according to claim 12 or 13, or a chimeric gene according to claim 14.

16. A host organism which contains a vector according to claim 15.

17. The host organism according to claim 16 which is a microorganism, a plant or animal cell.

18. A virus resistant host organism which carries in its genome a DNA sequence according to claim 11, a gene according to claim 12 or 13, or a chimeric gene according to claim 14.

19. The virus resistant host organism of claim 18 which is a microorganism, a plant or animal cell.

20. A virus resistant transgenic plant which is regenerated from a plant cell according to claim 19, and its virus resistant progeny.

21. A propagation material of a virus resistant plant according to claim 20.

22. A kit for the preparation of virus resistant, transgenic host organisms which contains a DNA sequence according to claim 11, a gene according to claim 12 or 13, a chimeric gene according to claim 14, or a vector according to 15.

23. The kit according to claim 22, wherein the virus resistant host organism is a plant cell, a plant or an animal cell.

24. An agent for treating viral infections which contains an RNA molecule according to any of claims 1 to 10, a DNA sequence according to claim 11, a gene according to claim 12 or 13, a chimeric gene according to claim 14, or a vector according to claim 15.
